# EUROPEAN PATENT APPLICATION

(11) **EP 4 534 556 A1**
(43) Date of publication of application: **09.04.2025**
(21) Application number: 23810909.4
(22) Date of filing: 17.05.2023
(51) Int. Cl.: C07K 16/28, C07K 19/00, C12N 15/13, G01N 33/68

(54) **CHIMERIC ANTIGEN RECEPTOR CONTAINING CLDN18.2 SINGLE DOMAIN ANTIBODY AND APPLICATION THEREOF**

(30) Priority: 27.05.2022 CN 202210594864
(71) Applicant: Suzhou Immunofoco Biotechnology Co., Ltd., Suzhou, Jiangsu 201210 (CN)
(72) Inventor: LI, Yantao, Suzhou, Jiangsu 215123 (CN); ZHONG, Yunpeng, Suzhou, Jiangsu 215123 (CN); HAO, Ruidong, Suzhou, Jiangsu 215123 (CN)
(74) Representative: Loyer & Abello
(86) International application number: PCT/CN2023/094648
(87) International publication number: WO 2023/226836

(57) **Abstract**

A chimeric antigen receptor containing a CLDN18.2 single domain antibody and an application thereof. The constructed chimeric antigen receptor is good in specificity, higher in safety in vivo use, and smaller in toxicity, and can provide a treatment solution for patients who suffer from advanced gastric cancer caused by high expression of a CLDN18.2 protein and currently undergo radiotherapy, chemotherapy, and surgery treatments.

## Description

### CROSS-REFERENCE TO RELATED APPLICATIONS

The present application claims priority to Patent Application No. CN2022105948645 filed on May 27, 2022, which is incorporated herein by reference in its entirety.

### TECHNICAL FIELD

The present disclosure relates to the technical field of biopharmaceuticals, and in particular, to a chimeric antigen receptor comprising a CLDN18.2 single domain antibody and use thereof.

### BACKGROUND

At present, the treatment principle for advanced gastric cancer is still comprehensive treatment mainly based on systemic chemotherapy, including systemic chemotherapy, surgical resection, radiotherapy, targeted therapy, immunotherapy, traditional Chinese medicine treatment, and the like. With the continuous optimization of chemotherapy drugs and chemotherapy regimens, the survival time of patients with advanced gastric cancer has been significantly prolonged, but the median survival time of the patients is still difficult to exceed 1 year.

In recent years, the development of immunotherapy has brought profound changes to the field of cancer treatment, especially with immune checkpoint therapies represented by CTLA-4 and PD1/PDL1 pathway inhibitors, and adoptive cell therapies represented by CAR-T. The adoptive cell therapies include TIL, NK, TCR-T, CAR-T/NK/NKT/TIL/Mø, and the like. Among them, the CAR-T therapy targeting CD19 has achieved excellent clinical efficacy in B-cell tumors, and in 2017, two CAR-T products were approved by the FDA for the treatment of B-cell leukemia or lymphoma.

Although targeted therapies and immune checkpoint inhibitors have been beneficial for specific populations, it is imperative to find other targets in advanced gastric cancer.

Claudins are a family of proteins that function to maintain tight junctions controlling intercellular molecular exchange. They are widely distributed in the stomach, pancreas and lung tissues, and can be used for diagnosis and treatment. The CLDN18.2 isoform is a stomach-specific isoform. Since Sahin discovered that CLDN18.2 is a highly selective molecule and is widely expressed only in cancer cells, it has become an ideal target. CLDN18.2 is typically embedded in the gastric mucosa and is substantially inaccessible to monoclonal antibodies in normal tissues. The occurrence of malignant tumors can result in the disruption of tight junctions, exposing the CLDN18.2 epitope on the surface of tumor cells and thus making it a specific target. Therefore, CLDN18.2 confers specificity to targeted therapies. It is expressed in various cancers, including 80% of gastrointestinal adenocarcinomas, 60% of pancreatic cancers, as well as biliary tract cancer, ovarian cancer, and lung cancer.

Currently, the global product types targeting CLDN18.2 include monoclonal antibodies, bispecific antibodies, CAR-T, and ADCs. Among them, the monoclonal antibodies have the largest number in development. The fastest progressing drug currently being developed for CLDN18.2 is IMAB362 (Zolbetuximab, claudiximab) developed by the German company Ganymed. IMAB362 is a chimeric human-mouse antibody that can specifically recognize and bind to the extracellular domain ECL1 of the claudin18.2 protein without binding to any other claudin family members (including claudin18.1). Currently, it has entered Phase III clinical trials.

Although CAR-T has made significant progress in hematological tumors, its efficacy in the treatment of solid tumors is poor. One of the key reasons is that solid tumors do not have as good targets as hematological tumors, and conventional CAR-T receptor sequences are derived from murine sources, which have a relatively strong affinity, so that CAR-T often leads to stronger on-target off-tumor effects and greater side effects in solid tumors. In addition, scFvs derived from murine sources have a relatively large molecular mass and relatively strong immunogenicity, and thus are more likely to form anti-antibodies in a patient, which results in CAR-T cells being rapidly cleared *in vivo* by the antibodies produced by the host, and failing to play a role in continuously alleviating the patient's tumor. This is also one of the reasons why CAR-T has relatively poor efficacy in solid tumors.

### SUMMARY OF THE INVENTION

One of the objectives of the present disclosure is to provide a chimeric antigen receptor comprising an extracellular domain comprising a CLDN18.2 single domain antibody, wherein amino acid sequences of heavy chain complementarity-determining regions of the single domain antibody are set forth in SEQ ID NOs: 1-3.

The present disclosure further relates to a nucleic acid and a vector related to the chimeric antigen receptor as described above.

The present disclosure further provides an immune cell expressing the chimeric antigen receptor as described above.

The present disclosure further relates to a pharmaceutical composition comprising the immune cell as described above.

The present disclosure further relates to use of the immunoglobulin variable domain or the immune cell as described above in the preparation of a medicament for killing CLDN18.2-positive tumor cells.

The inventors of the present disclosure have surprisingly found that the single domain antibody selected in the present disclosure can achieve excellent technical effects when constructed as a chimeric antigen receptor. The constructed chimeric antigen receptor has good specificity, offers higher safety and lower toxicity when used *in vivo,* and can provide a potentially better treatment solution for patients who suffer from advanced gastric cancer with high expression of a CLDN18.2 protein and currently undergo radiotherapy, chemotherapy, and surgery treatments

### BRIEF DESCRIPTION OF THE DRAWINGS

In order to more clearly illustrate the specific embodiments of the present disclosure or technical solutions in the prior art, the drawings used in the descriptions of the specific embodiments or the prior art are briefly introduced below. It is obvious that the drawings in the following description are only illustrative of some embodiments of the present disclosure. On the basis of these drawings, other drawings can be obtained by those of ordinary skill in the art without creative efforts.

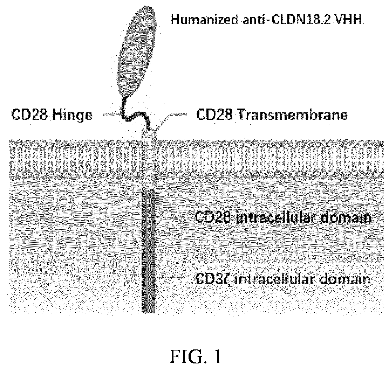

FIG. 1 shows a schematic diagram of the IMC002 CAR-T structure;
FIG. 2 shows the IHC staining of CLDN18.2 on normal human tissues;
FIG. 3 shows the expression of CLDN18.2 in human gastric cancer tissues;
FIG. 4 shows the expression of CLDN18.2 on different target cells;
FIG. 5 shows an assay for CAR expression;
FIG. 6 shows an assay for the killing ability of CAR-T against target cells;
FIG. 7 shows an assay for the IFN-γ secretion ability after killing of target cells by CAR-T;
FIG. 8 shows the results of a cytotoxicity assay for 21047 and other CAR-T cells;
FIG. 9 shows the results of a cytotoxicity assay for 21050 CAR-T cells;
FIG. 10 shows the inhibition of NUGC4-luc subcutaneous graft tumor growth in mice by 21047 and other CAR-T cells;
FIG. 11 shows the binding of 21047 to primary cells (lines);
FIG. 12 shows the killing ability of 21047 CAR-T cells against human and mouse lung tissues;
FIG. 13 shows the killing ability of 21047 and other CAR-T cells against primary human cells/tissues;
FIG. 14 shows the functional verification of several variants of 21047 CAR-T after replacement of different domains.

### DETAILED DESCRIPTION

References for embodiments of the present disclosure are provided in detail by means of one or more examples below. Each of the examples is provided for illustrating rather than limiting the present disclosure. Actually, it is obvious to those skilled in the art that various modifications and variations can be made to the present disclosure without departing from the scope or spirit of the present disclosure. For example, a feature stated or described as part of one embodiment may be used in another embodiment to produce a further embodiment.

Unless otherwise stated, all terms (including technical and scientific terms) used to disclose the present disclosure have the same meaning as would normally be understood by those of ordinary skill in the art. With further guidance, subsequent definitions are used to better understand the teachings of the present disclosure. The terms used in the specification of the present disclosure are for the purpose of describing the specific examples only and are not intended to limit the present disclosure.

The term "and/or" or "or/and" as used herein includes any one of two or more relevant listed items, and any and all combinations of relevant listed items. The any and all combinations include a combination of any two or more or all of the relevant listed items. It will be appreciated that in the present application, when at least three items are connected by at least two combinations of conjunctions selected from "and/or" and "or/and", the technical solution undoubtedly encompasses the instance where both are connected by logical "and" and the instance where both are connected by logical "or". For example, "A and/or B" includes three parallel instances, i.e., A, B, and A+B. For another example, a technical solution of "A, and/or, B, and/or, C, and/or, D" includes any one item of A, B, C, and D (the instance where the items are connected by logic "or"), and also includes any and all of the combinations of A, B, C, and D. That is, combinations of any two or three of A, B, C, and D, as well as the combination of A, B, C, and D (the instance where the items are connected by logic "and") are included.

The terms "comprise", "contain", and "include" as used herein are synonyms, and are inclusive or open-ended but not exclusive of additional or uncited members, elements, or procedures.

A numeral range represented by endpoints as used herein includes all values and fractions within the range, as well as the endpoint.

In the present disclosure, descriptions involving "various", "more", or the like, unless otherwise defined, refer to a quantity of 2 or more.

In the present disclosure, the technical features described in an open-ended manner include closed-ended technical solutions consisting of the enumerated features, and also include open-ended technical solutions containing the enumerated features.

The present disclosure relates to a chimeric antigen receptor, comprising an extracellular domain comprising a CLDN18.2 single domain antibody, wherein amino acid sequences of heavy chain complementarity-determining regions of the single domain antibody are set forth in SEQ ID NOs: 1-3.

As used herein, the "chimeric antigen receptor (CAR)" refers to a fusion protein that contains an extracellular domain capable of binding to an antigen, a transmembrane domain derived from a polypeptide different from the polypeptide where the extracellular domain is derived, and at least one intracellular domain. The "chimeric antigen receptor (CAR)" is sometimes referred to as "chimeric receptor" or "chimeric immune receptor (CIR)". The term "extracellular domain capable of binding to an antigen" refers to any oligopeptide or polypeptide capable of binding to a specific antigen. The "intracellular domain" refers to any oligopeptide or polypeptide known to function in cells as a domain that transmits signals to activate or inhibit a biological process.

A single domain antibody refers to a type of antibody that lacks the antibody light chain and only has the heavy chain variable region. Because of its small molecular weight, it is also known as a nanobody [note: Nanobody^{™}, Nanobodies^{™}, and Nanoclone^{™} are trademarks of Ablynx N.V.]. A single domain antibody may or may not have a constant region. In some contexts, it can be used interchangeably with the V_{H}H segment.

A single domain antibody can be obtained by activating the immune system of *Camelus dromedarius, Camelus bactrianus, Vicugna pacos, Lama glama,* or shark with a specific antigen and then isolating an mRNA producing a heavy-chain antibody. The single domain antibody has a constant region, which may be derived from *Camelus dromedarius, Camelus bactrianus, Vicugna pacos, Lama glama,* or shark. In some embodiments, the single domain antibody has constant regions CH1-CH5 (such as IgNAR). In some embodiments, the single domain antibody has constant regions CH2 and CH3 (hcIgG).

As further described herein, the amino acid sequence and structure of the single domain antibody (however not limited thereto) comprise four framework regions or "FRs", which are separated by three complementarity-determining regions or "CDRs".

In some embodiments, the single domain antibody is humanized.

The term "humanized antibody", also known as a CDR-grafted antibody, refers to an antibody produced by grafting CDR sequences of first animal origin (e.g., murine, rabbit, etc.) into a human antibody variable region framework, i.e., a different type of human germline antibody framework sequence. Such an antibody can overcome the heterogeneous response induced by the chimeric antibody because of carrying a large amount of protein components of first animal origin. Such framework sequences can be obtained from public DNA databases or published references that include germline antibody gene sequences. For example, germline DNA sequences of genes of human heavy and light chain variable regions can be found in the "VBase" human germline sequence database (www.mrccpe.com.ac.uk/vbase), as well as in Kabat, E. A. et al., 1991, Sequences of Proteins of Immunological Interest, 5th edition. To avoid the decrease in activity caused by the decrease in immunogenicity, the framework sequence in the human antibody variable region can be subjected to minimum reverse mutation or back mutation to maintain activity. The humanized antibody of the present disclosure also includes humanized antibodies formed by further affinity maturation of the CDRs by phage display. Human antibody variable region frameworks are designed and selected, for example, wherein the heavy chain FR sequence on the antibody heavy chain variable region is derived from a combined sequence of the human germline heavy chain IGHV1-18*01 and hjh6.1, or a combined sequence of the human germline heavy chain IGHV1-3*01 and hjh6.1; the light chain FR sequence on the antibody light chain variable region is derived from a combined sequence of the human germline light chain IGKV1-39*01 and hjk4.1. To avoid the decrease in activity caused by the decrease in immunogenicity, the human antibody variable region can be subjected to minimum reverse mutation to maintain activity.

In some embodiments, an amino acid sequence of the single domain antibody is set forth in SEQ ID NO: 4.

Modified forms of the single domain antibody are also within the protection scope of the present disclosure, such as those modified by covalent attachment of polyethylene glycol or other suitable polymers. Variants of the single domain antibody are also within the scope of the present disclosure, wherein the variants of CDR1 to CDR3 may each comprise a mutation of up to 3 amino acids (e.g., replacement, deletion or addition of 1, 2, or 3 amino acids, or any combination thereof) as compared to any one of combinations of the complementarity-determining regions set forth in SEQ ID NOs: 1-3; preferably, the mutation is a conservative mutation. The "conservative replacement" refers to replacement of an amino acid in a protein with another amino acid that has similar characteristics (e.g., charge, side-chain size, hydrophobicity/hydrophilicity, backbone conformation and rigidity, etc.), so that changes can be frequently made without altering the biological activity of the protein.

Replacements that are generally considered conservative replacements include the mutual replacement in the aliphatic amino acids Ala, Val, Leu, and Ile; the interchange of the hydroxyl residues Ser and Thr; the exchange of the acidic residues Asp and Glu; the replacement between the amide residues Asn and Gln; the exchange of the basic residues Lys and Arg; and the replacement between the aromatic residues Phe and Tyr. Those skilled in the art know that, generally speaking, a single amino acid replacement in a non-essential region of a polypeptide does not substantially alter the biological activity (see, e.g., Watson et al. (1987) Molecular Biology of the Gene, The Benjamin/Cummings Pub. Co., p224, (4th edition)). In addition, the replacement of structurally or functionally similar amino acids is unlikely to disrupt the biological activity.

In some embodiments, the chimeric antigen receptor comprises a hinge region, a transmembrane region, and an intracellular signaling region.

As used herein, the "region" or "domain" in the chimeric antigen receptor refers to a region of a polypeptide that can be folded into a specific structure independently of other regions. Such "regions" or "domains" may be sequences of murine origin or other animal origins, preferably of human origin. In addition, the "region" or "domain", unless specifically distinguished or emphasized, should be understood as a well-known sequence, which may be the full length or an active segment thereof.

In some embodiments, the hinge region is selected from a hinge region of CD8, CD28, IgG1, IgG4, 4-1BB, ICOS, OX40, CD40, CD80, or CD7, or a CH3 or CH2-CH3 constant region. Preferably, an amino acid sequence of the CD28 hinge region is set forth in SEQ ID NO: 6 or SEQ ID NO: 7, and an amino acid sequence of the CD8 hinge region is set forth in SEQ ID NO: 8.

The transmembrane region is selected from one of the α, β, or ζ chain of a T-cell receptor, CD28, CD3ε, CD45, CD4, CD5, CD8, CD9, CD16, CD22, CD33, CD37, CD64, CD80, CD86, CD134, CD137, CD154, KIRDS2, OX40, CD2, CD27, LFA-1 (CD11a, CD18), ICOS (CD278), 4-1BB (CD137), GITR, CD40, BAFFR, HVEM (LIGHTR), SLAMF7, NKp80 (KLRF1), CD160, CD19, IL2Rβ, IL2Rγ, IL7Rα, ITGA1, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, DNAM1 (CD226), SLAMF4 (CD244 and 2B4), CD84, CD96 (Tactile), CEACAM1, CRTAM, Ly9 (CD229), CD160 (BY55), PSGL1, CD100 (SEMA4D), SLAMF6 (NTB-A and Ly108), SLAM (SLAMF1, CD150, and IPO-3), BLAME (SLAMF8), SELPLG (CD162), LTBR, PAG/Cbp, NKp44, NKp30, NKp46, NKG2D, and NKG2C. In some embodiments, the transmembrane region is preferably a transmembrane region of CD8a, CD28, CD4, ICOS, CD7, CD2, CD80, CD40, OX40, CD27, LFA-1, 4-1BB, ICOS, CD3ζ, or CD3ε; more preferably, an amino acid sequence of the CD28 transmembrane region is set forth in SEQ ID NO: 9, and an amino acid sequence of the CD8 transmembrane region is set forth in SEQ ID NO: 10.

In some embodiments, the intracellular signaling region comprises a CD3ζ signaling domain, with the preferred amino acid sequence set forth in SEQ ID NO: 11.

In some embodiments, the intracellular signaling region further comprises a protein or an intracellular signaling region (or co-stimulatory region) thereof selected from those shown in the following table; in some embodiments, the intracellular signaling region further comprises one or more selected from the following proteins or intracellular signaling regions thereof: CD28, 4-1BB, OX40, ICOS, CD27, MYD88, KIR2DS2, DAP10, DAP12, CD3ζ, TLRs, CD2, LFA-1, CD8α, CD40, CD80, and CD3ε.

Exemplary intracellular signaling regions useful in the present disclosure and corresponding exemplary sequences are shown in the following table:

| Signaling region name | UniProt accession No. | Amino acid position |
|---|---|---|
| 2B4 | Q9BZW8 | 251-370 |
| 4-1BB | Q07011 | 214-255 |
| BTLA | Q7Z6A9 | 179-289 |
| CD2 | P06729 | 236-351 |
| CD22 | P20273 | 707-847 |
| CD27 | P26842 | 213-260 |
| CD28 | P10747 | 180-220 |
| CD30 | P28908 | 407-595 |
| CD3d | P04234 | 127-171 |
| CD3d -ITAM | P04234 | 138-166 |
| CD3e | P07766 | 153-207 |
| CD3e -ITAM | P07766 | 178-205 |
| CD3g | P09693 | 138-182 |
| CD3g -ITAM | P09693 | 149-177 |
| CD3z | P20963 | 52-164 |
| CD3z ITAM1 | P20963 | 61-89 |
| CD3z ITAM2 | P20963 | 100-128 |
| CD3z ITAM3 | P20963 | 131-159 |
| CD4 | P01730 | 419-458 |
| CD40 | P25942 | 216-277 |
| CD79a | P11912 | 166-226 |
| CD79a ITAM | P11912 | 177-205 |
| CD79b | P40259 | 181-229 |
| CD79b ITAM | P40259 | 185-213 |
| CD84 | Q9UIB8 | 247-345 |
| CD8a | P01732 | 204-235 |
| CD8b | P10966 | 192-210 |
| CRACC | Q9NQ25 | 248-335 |
| CRTAM | O95727 | 309-393 |
| CTLA-4 | P16410 | 183-223 |
| DAP10 | Q9UBK5 | 70-93 |
| DAP12 | 043914 | 62-113 |
| DAP12 ITAM | 043914 | 80-108 |
| DNAM-1 | Q15762 | 276-336 |
| DR3 | Q93038 | 221-417 |
| FCER1G | P30273 | 45-86 |
| FCER1G-ITAM | P30273 | 54-82 |
| FCGR1A | P12314 | 314-374 |
| FCGR2A | P12318 | 241-317 |
| FCGR2B | P31994 | 241-310 |
| FCGR3A | P08637 | 230-254 |
| FCRL1 | Q96LA6 | 329-429 |
| FCRL2 | Q96LA5 | 423-508 |
| FCRL3 | Q96P31 | 595-734 |
| FCRL4 | Q96PJ5 | 409-515 |
| FCRL5 | Q96RD9 | 873-977 |
| FCRL6 | Q6DN72 | 329-434 |
| GITR | Q9Y5U5 | 184-241 |
| HVEM | Q92956 | 224-283 |
| ICOS | Q9Y6W8 | 162-199 |
| KIR2DL1 | P43626 | 265-348 |
| KIR2DL2 | P43627 | 265-348 |
| KIR2DL3 | P43628 | 266-341 |
| KIR2DL4 | Q99706 | 264-377 |
| KIR2DL5A | Q8N109 | 260-375 |
| KIR2DL5B | Q8NHK3 | 260-375 |
| KIR3DL1 | P43629 | 361-444 |
| KIR3DL2 | P43630 | 361-455 |
| KIR3DL3 | Q99706 | 264-377 |
| LAG3 | P18627 | 472-525 |
| LAT | O43561 | 28-262 |
| LILRB1 | Q8NHL6 | 483-650 |
| LILRB2 | Q8N423 | 483-598 |
| Ly9 | Q9HBG7 | 477-655 |
| NKG2A | P26715 | 1-70 |
| NKG2C | P26717 | 1-70 |
| NKG2D | P26718 | 1-51 |
| NKp30 | O14931 | 157-201 |
| NKp44 | O95944 | 214-276 |
| NKp46 | O76036 | 280-304 |
| OX40 | P43489 | 236-277 |
| PD-1 | Q15116 | 192-288 |
| PILRB | Q9UKJ0 | 213-227 |
| SIRPa | P78324 | 395-504 |
| SLAMF1 | Q13291 | 259-335 |
| SLAMF6 | Q96DU3 | 248-331 |
| SLAMF7 | Q9NQ25 | 248-355 |
| TIGIT | Q495A1 | 163-244 |
| TIM1 | Q96D42 | 317-364 |
| TIM3 | Q8TDQ0 | 224-301 |
| TLR1 | Q15399 | 602-786 |
| TLR10 | Q9BXR5 | 598-811 |
| TLR2 | O60603 | 610-784 |
| TLR3 | O15455 | 726-904 |
| TLR4 | O00206 | 653-839 |
| TLR5 | O60602 | 661-858 |
| TLR6 | Q9Y2C9 | 608-796 |
| TLR7 | Q9NYK1 | 861-1049 |
| TLR8 | Q9NR97 | 849-1031 |
| TLR9 | Q9NR96 | 840-1032 |

In some embodiments, the intracellular signaling region further comprises CD28 having the amino acid sequence set forth in SEQ ID NO: 12; or OX40 having the amino acid sequence set forth in SEQ ID NO: 13; or 4-1BB having the amino acid sequence set forth in SEQ ID NO: 14; or ICOS having the amino acid sequence set forth in SEQ ID NO: 15.

In some embodiments, an amino acid sequence of the chimeric antigen receptor is set forth in SEQ ID NO: 5.

Variants of SEQ ID NOs: 4/5 are also within the protection scope of the present disclosure, and the variants may have, for example, 80%, 81%, 82%, 83%, 84%, 85%, 86%, 87%, 88%, 89%, 90%, 91%, 92%, 93%, 94%, 95%, 96%, 97%, 98% or 99% identity to the sequences set forth in SEQ ID NOs: 4/5. The variants may also have conservative replacements as described above.

According to another aspect of the present disclosure, the present disclosure further relates to an isolated nucleic acid capable of expressing the chimeric antigen receptor as described above.

Herein, the nucleic acid includes variants thereof with conservative replacements (e.g., degenerate codon replacements) and complementary sequences, as well as variants that are codon-optimized for more efficient expression in the desired host cells. Nucleic acids are typically RNA or DNA, including genes, cDNA molecules, mRNA molecules, and fragments thereof such as oligonucleotides. The nucleic acid molecule may be single-stranded or double-stranded, but is preferably double-stranded DNA. A nucleic acid is "operably linked" when it is placed into a functional relationship with another nucleic acid sequence. For example, a promoter or enhancer is operably linked to a coding sequence if it affects the transcription of the coding sequence. When it is incorporated into a vector, a DNA nucleic acid is preferably used.

The present disclosure further relates to a vector comprising the nucleic acid as described above.

The term "vector" refers to a nucleic acid vehicle into which a polynucleotide can be inserted. When a vector allows the expression of the protein encoded by the inserted polynucleotide, the vector is referred to as an expression vector. The vector can be introduced into a host cell by transformation, transduction, or transfection, such that the genetic substance elements carried by the vector can be expressed in the host cell. Vectors are well-known to those skilled in the art, including but not limited to: plasmids; phagemids; CRISPR/CAS plasmids; cosmids; artificial chromosomes such as yeast artificial chromosome (YAC), bacterial artificial chromosome (BAC), or P1-derived artificial chromosome (PAC); phages such as λ phages or M13 phages; and animal viruses, etc. Animal viruses that can be used as vectors include, but are not limited to, retroviruses, lentiviruses, adenoviruses, adeno-associated viruses, herpes viruses (e.g., herpes simplex virus), poxviruses, baculoviruses, papillomaviruses, and papovaviruses (e.g., SV40). In some embodiments, the vector of the present disclosure comprises regulatory elements commonly used in genetic engineering, such as enhancers, promoters, internal ribosome entry sites (IRESs), and other expression control elements (e.g., transcription termination signals, or polyadenylation signals and poly U sequences, etc.).

The present disclosure further relates to an immune cell expressing the chimeric antigen receptor as described above. The immune cell is, for example, one or more of a T cell, a B cell, an NK cell, a macrophage, a dendritic cell, and the like.

In some embodiments, the immune cell is a T cell.

The T cell may be of a subclass well-known in the art, such as one or more of a helper T cell, a cytotoxic T cell, a memory T cell, a regulatory T cell, a MAIT cell, an NKT cell, and a γδ T cell.

According to yet another aspect, the present disclosure further relates to a pharmaceutical composition comprising the immune cell as described above.

The pharmaceutical composition may further comprise a pharmaceutically acceptable carrier. As used herein, the "pharmaceutically acceptable carrier" includes any material that, when combined with an active component, allows the component to maintain biological activity and does not react with the subject's immune system. Examples include, but are not limited to, any of standard pharmaceutical carriers (e.g., phosphate-buffered saline solutions, water, emulsions (such as oil/water emulsions)), and various types of wetting agents. Exemplary diluents for aerosol or parenteral administration are phosphate-buffered saline (PBS) or normal saline (0.9%). Compositions containing such carriers are formulated using well-known conventional methods (see, e.g., Remington's Pharmaceutical Sciences, 18th edition, A. Gennaro, eds., Mack Publishing Co., Easton, PA, 1990; and Remington, The Science and Practice of Pharmacy, 21st edition, Mack Publishing, 2005).

According to still another aspect of the present disclosure, the present disclosure further relates to use of the immune cell as described above in the preparation of a medicament for killing CLDN18.2-positive tumor cells.

In some embodiments, the medicament is used for preventing or treating gastric cancer.

According to still another aspect of the present disclosure, the present disclosure further relates to a method for treating a tumor in a patient in need, comprising administering to the patient a therapeutically effective amount of the immune cell or the pharmaceutical composition as described above.

The tumor is preferably a solid tumor; in the present disclosure, the "solid tumor" include tumors derived from lesions at any of the following: bones, bone junctions, muscles, lungs, tracheae, heart, spleen, arteries, veins, capillaries, lymph nodes, lymph vessels, lymph fluid, mouth, pharynx, esophagus, stomach, duodenum, small intestine, colon, rectum, anus, appendix, liver, gallbladder, pancreas, parotid glands, sublingual glands, urinary kidneys, ureters, bladder, urethra, ovaries, fallopian tubes, uterus, vagina, vulva, scrotum, testicles, vas deferens, penis, eyes, ears, nose, tongue, skin, brain, brainstem, medulla oblongata, spinal cord, cerebrospinal fluid, nerves, thyroid, parathyroid glands, adrenal glands, pituitary gland, pineal gland, pancreatic islet, thymus, gonads, and sublingual glands.

In some embodiments, tumor cells in the tumor are CLDN18.2-positive.

In some embodiments, the tumor is gastric cancer, pancreatic cancer, or esophageal cancer.

It should be understood that the contemplated treatment method may further include administration of an additional immunotherapeutic entity, particularly preferably an immunotherapeutic entity, including viral cancer vaccines (e.g., adenoviral vectors encoding cancer-specific antigens), bacterial cancer vaccines (e.g., non-pyrogenic *Escherichia coli* expressing one or more cancer-specific antigens), yeast cancer vaccines, N-803 (also known as ALT-803, ALTOR BioScience), and antibodies (e.g., those binding to tumor-associated antigens or patient-specific tumor neoantigens), stem cell grafts (e.g., allogeneic or autologous), and tumor-targeting cytokines (e.g., NHS-IL12 and IL-12 conjugated with tumor-targeting antibodies or fragments thereof).

The "patient" is a mammal, including, but not limited to, humans, monkeys, pigs, and other farm animals, sport animals, pets, primates, horses, dogs, cats, pandas, rodents (including mice, rats, and guinea pigs), and the like.

The embodiments of the present disclosure will be described in detail below with reference to the examples. It should be understood that these examples are for illustrative purposes only and are not intended to limit the scope of the present disclosure. Procedures without specified conditions in the following examples are preferably conducted with reference to the guidelines provided in the present disclosure, in accordance with experimental manuals or conventional conditions in the art, with reference to other experimental procedures known in the art, or in accordance with conditions recommended by the manufacturers.

In the specific examples below, the measurement parameters for raw material components may have slight deviations within the range of measurement accuracy, unless otherwise specified. For the temperature and time parameters, acceptable deviations caused by instrument testing accuracy or operational accuracy are permitted.

### Examples

### 1. Study on tissue expression of the CLDN 18.2 target

The human CLDN18 gene has two alternative first exons, resulting in two distinct protein isoforms, CLDN18.1 and CLDN18.2 (there are only 8 amino acid differences in extracellular region 1 between CLDN18.1 and CLDN18.2). The two isoforms have different lineage functions: CLDN18.1 is expressed predominantly in lung tissue, whereas CLDN18.2 is mainly specific to the stomach. The CLDN18.2 protein, as a highly selective gastric lineage marker, is expressed in short-cycle differentiated cells and is not expressed in the stem cell region of the gastric mucosa.

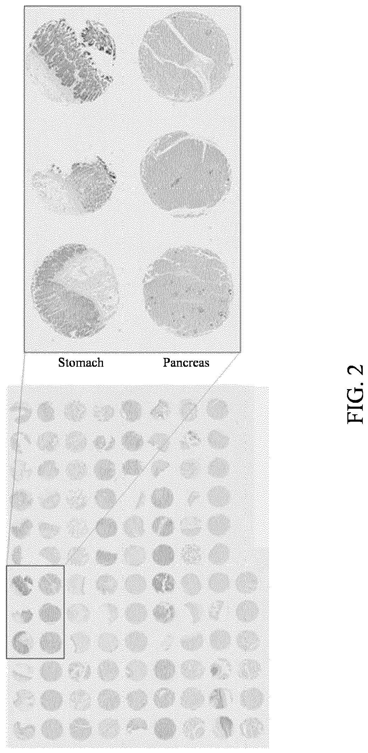

In order to study the distribution of CLDN18.2 in normal human tissues and organs, we used a CLDN18.2-specific IHC antibody to detect the expression of CLDN18.2 in major normal human organs. The results are shown in FIG. 2, indicating that CLDN18.2 was mainly expressed in gastric tissues, with a small amount of expression in pancreatic tissues, and was almost not expressed in other tissues.

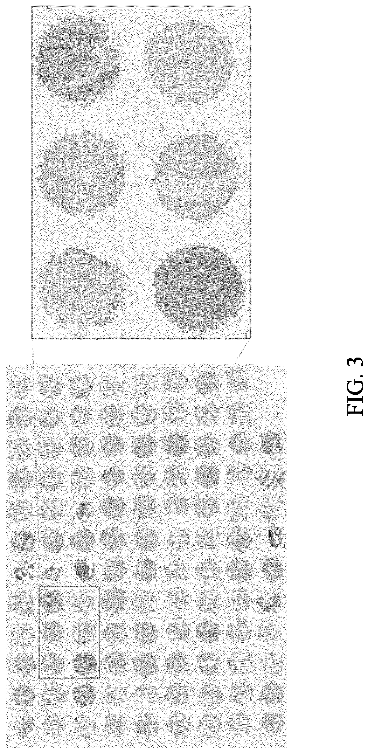

In order to further study the expression of CLDN18.2 in gastric tumor tissues, we used a CLDN18.2-specific antibody to detect the expression of CLDN18.2 in human gastric tumor tissues. The detection results are shown in FIG. 3, indicating that CLDN18.2 was highly expressed on the membranes of over 30% of gastric cancer cells and was highly expressed on glandular epithelial cells of normal gastric tissues and gastric paracancerous tissues.

### 2. In vitro functional study of CLDN18.2 CAR-T cells

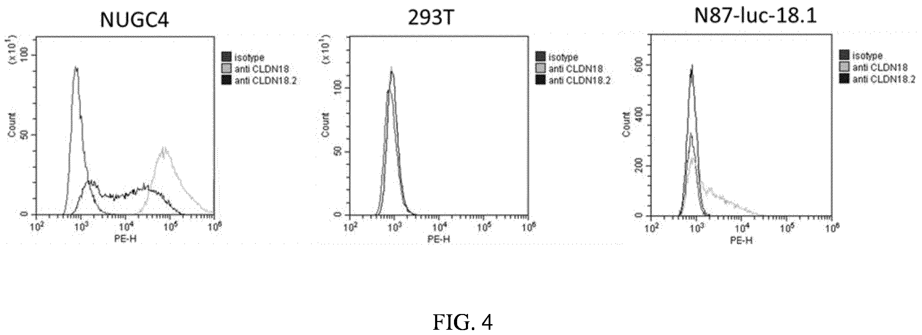

We firstly studied the *in vitro* target cell killing and cytokine secretion abilities of CLDN18.2 CAR-T cells guided by different extracellular regions, such as 21008, 21009, 21011, 21047, and 21050 (with sequences set forth in SEQ ID NOs: 16, 17, 18, 4, and 19, respectively); the fragments used for the other moieties of their CARs were the same as SEQ ID NO: 5, i.e., the fragments obtained by splicing SEQ ID NOs: 6-8. Since CLDN18.2 differs from its other isoform CLDN18.1 by only 7 amino acids in the extracellular region, in order to study whether CAR-T could recognize the differences between CLDN18.2 and CLDN18.1, we firstly constructed cell lines expressing CLDN18.2 and CLDN18.1, and identified the expression levels of CLDN18.2 and CLDN18.1 on different target cells. The results of flow cytometry (FCM) staining are shown in FIG. 4, indicating that the cells of the gastric cancer cell line NUGC4 highly expressed CLDN18.2, N87-luc-18.1 moderately expressed CLDN18.1 and did not express CLDN18.2, and 293T cells did not express either CLDN18.1 or CLDN18.2.

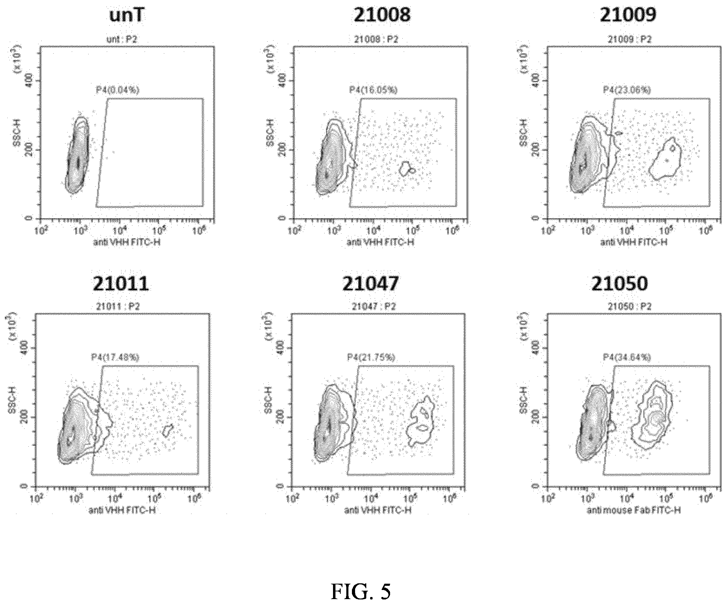

In order to study whether CAR-T cells such as 21008, 21009, 21011, 21047, and 21050 were specifically activated by CLDN18.2 target cells, we constructed several types of CAR-T cells and identified CAR expression. The results are shown in FIG. 5.

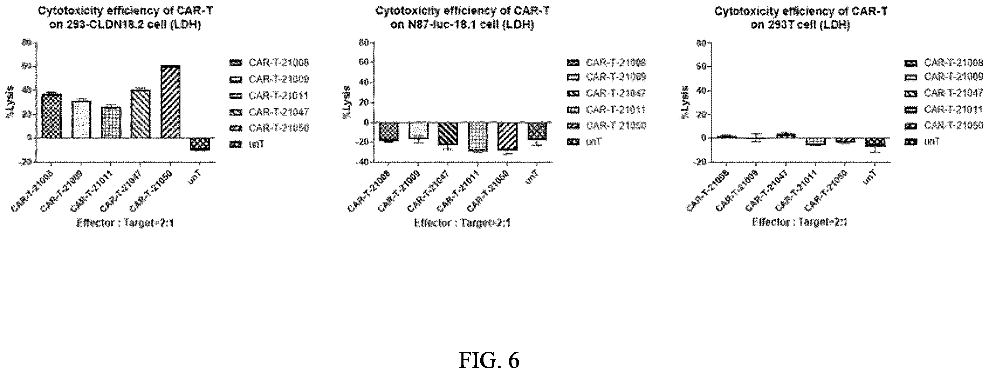

The CAR-T cells constructed above were co-incubated with target cells, and the ability of effector cells to lyse the target cells was assayed using the LDH method. The results are shown in FIG. 6, indicating that all five types of CAR-T cells tested could significantly kill the CLDN18.2-positive target cells NUGC4, while not killing the negative target cells N87-luc-CLDN18.1 and 293T, suggesting that the five types of CAR-T cells could specifically recognize CLDN18.2 and not recognize CLDN18.1.

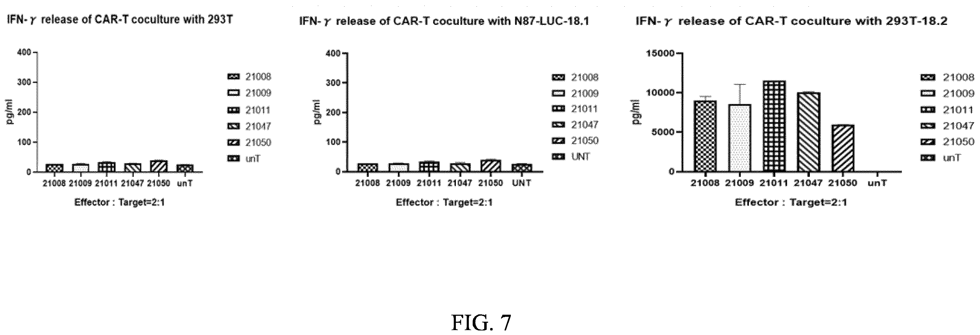

After co-incubation of the CAR-T cells constructed above with target cells, the release of the cytokine IFN-γ was assayed. The results are shown in FIG. 7. After co-incubation of five types of CAR-T cells, including 21047, with the CLDN18.2-positive target cells NUGC4, the IFN-γ secretion ability was activated, and IFN-γ secretion levels reached above 5000 pg/mL; in contrast, after co-incubation with the negative target cells N87-luc-18.1 and 293T, the cytokine secretion was below 100 pg/mL. This indicates that the tested CAR-T cells could be specifically activated by NUGC4, but could not be activated after co-incubation with the negative target cells N87-luc-18.1 and 293T;

### 3. In vivo safety study of single administration of CLDN18.2 CAR-T cells

We further studied the *in vivo* safety of five CAR-T cells: 21008, 21009, 21011, 21047, and 21050.

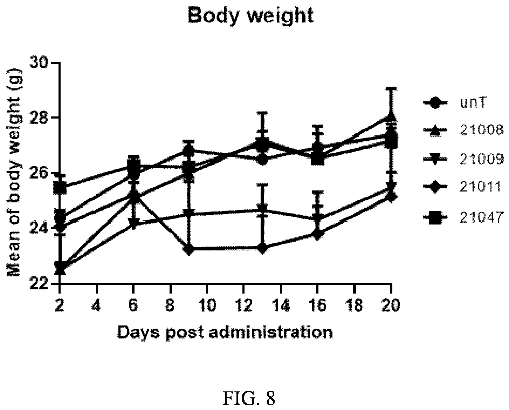

In an *in vivo* experiment, we compared four CAR-T cells: 21008, 21009, 21011, and 21047. We performed a single CAR-T administration in an NSG mouse model, at a dose of 5 × 10⁶ cells/mouse. After infusion, the mice were observed for status and weighed twice a week. During the experiment, no obvious abnormalities were observed in the mice of the 21047 CAR-T 5 M group, and they did not experience significant weight loss during the study (FIG. 8); in contrast, the 21011 group had a noticeable weight loss course, followed by gradual recovery. At the end of the study, we collected the vital organs of each mouse for H&E staining. The pathological results are shown in the following table:

| Group | ID | Brain | Heart | Liver | Spleen | Lung | Kidney | Digestive tract |
|---|---|---|---|---|---|---|---|---|
| unT | 114 | 0 | 0 | 0 | 0 | 0 | 0 | 0 |
| | 115 | 0 | 0 | 0 | 0 | 0 | 0 | 0 |
| 21008 | 14 | 0 | 0 | 1+0+0 | 0 | 2+0+0 | 0 | 0 |
| | 16 | 0 | 0 | 1+0+0 | 0 | 1+0+0 | 0 | 0 |
| 21009 | 18 | 0 | 0 | 1+3+0 | 0 | 1+0+0 | 0 | 0 |
| | 17 | 0 | 0 | 1+0+0 | 0 | 1+0+0 | 0 | 0 |
| 21010 | 21 | 0 | No slicing | 0+0+1 | 0 | No slicing | 0 | 0 |
| | 22 | 0 | 0 | 0+0+1 | No slicing | 3+0+0 | 0 | 0 |
| 21011 | 97 | 0 | 0 | 0+0+1 | 0 | 3+0+0 | 0 | 0 |
| | 98 | 0 | 0 | 1+0+0 | 0 | 1+0+0 | 0 | 0 |
| 21047 | 95 | 0 | 0 | 0 | 0 | 0 | 0 | 0 |
| | 93 | 0 | 0 | 0 | 0 | 0 | 0 | 0 |
| Conclusion: | | No significant differences were observed among the groups | No significant differences were observed among the groups | There were different manifestations, including proliferation of small bile ducts, cholestasis, and focal steatosis; in severe cases, hepatocellular edema was observed. | No significant differences were observed among the groups | The lesions were primarily observed in the lung, with obvious alveolar epithelial hyperplasia and consolidation. There was an impairment of effective blood-gas exchange in the alveolar spaces; in some cases, some cases were | No significant differences were observed among the groups | No significant differences were observed among the groups |
| | | | | | | accompanied by alveolar hemorrhage. | | |
| Scoring criteria | Lung: glandular epithelial hyperplasia and consolidation + acute necrosis + red blood cell effusion (all rated as none = 0, mild = 1, moderate = 2, severe = 3) | | | | | | | |
| | Digestive tract: | | | | | | | |
| | Kidney: | | | | | | | |
| | Liver: cholestasis + hepatocellular edema and degeneration + proliferation of small bile ducts (all rated as none = 0, mild = 1, moderate = 2, severe = 3) | | | | | | | |
| | Spleen: | | | | | | | |
| | Heart: | | | | | | | |
| | Brain: | | | | | | | |
| Summary: | | | | | | | | |
| The lesions were primarily caused by lung damage, with liver damage as a secondary cause. Microscopically, lung lesions showed proliferation of glandular epithelial cells, increased nuclear division, and alveolar space consolidation, which could cause impaired blood-gas barrier exchange and respiratory failure; liver lesions were manifested by proliferation of small bile ducts, cholestasis, or focal hepatocellular edema in the liver, and in some cases, ballooning degeneration of hepatocytes (severe edema). | | | | | | | | |

Except for the 21047 and unT groups, mice in the 21008, 21009, and 21011 treatment groups all showed some degree of tissue damage or obvious histological changes in their organs (including the brain, heart, liver, lung, kidney, and stomach). In summary, the 21047 CAR-T cells demonstrated good safety in the mouse model.

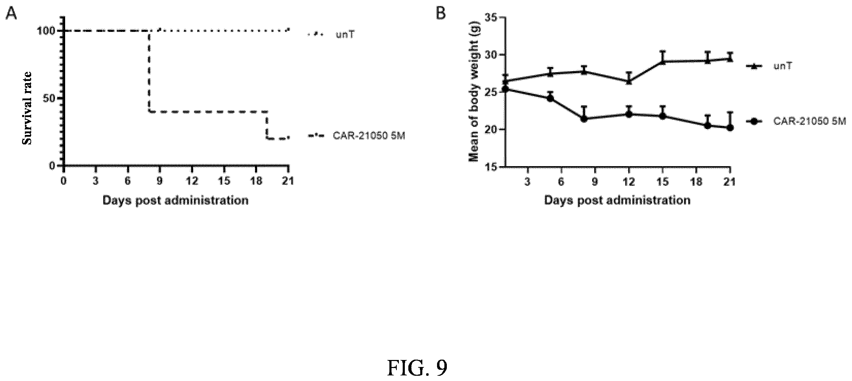

In another experiment, we compared the safety of 21050 and unT with a single CAR-T administration in an NSG mouse model, at a dose of 5 × 10⁶ cells/mouse. After infusion, the mice were observed for status and weighed twice a week. During the experiment, obvious abnormalities were observed in the mice of the 21050 CAR-T 5 M group, and they experienced significant weight loss and death during the study (FIGs. 9A/B), suggesting that 21050 also exhibited significant toxicity in the mouse model.

### 4. In vivo efficacy study of CLDN18.2 CAR-T cells

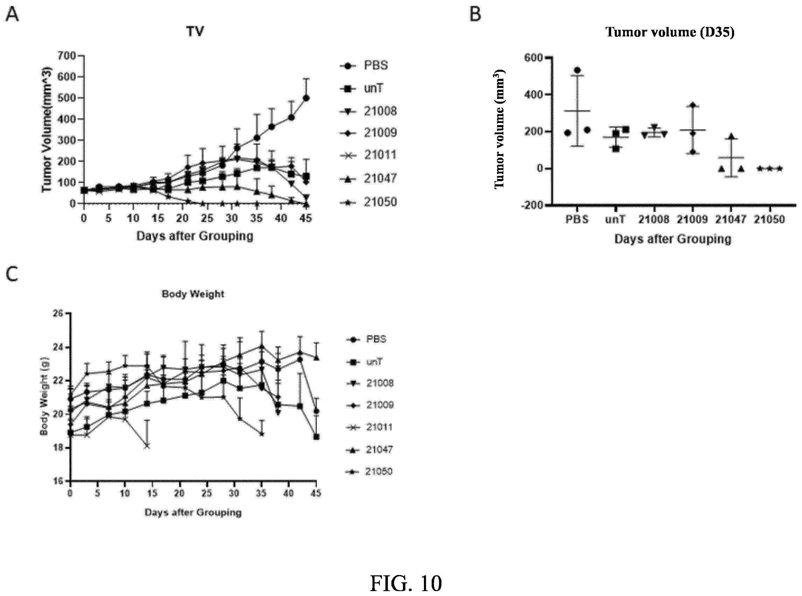

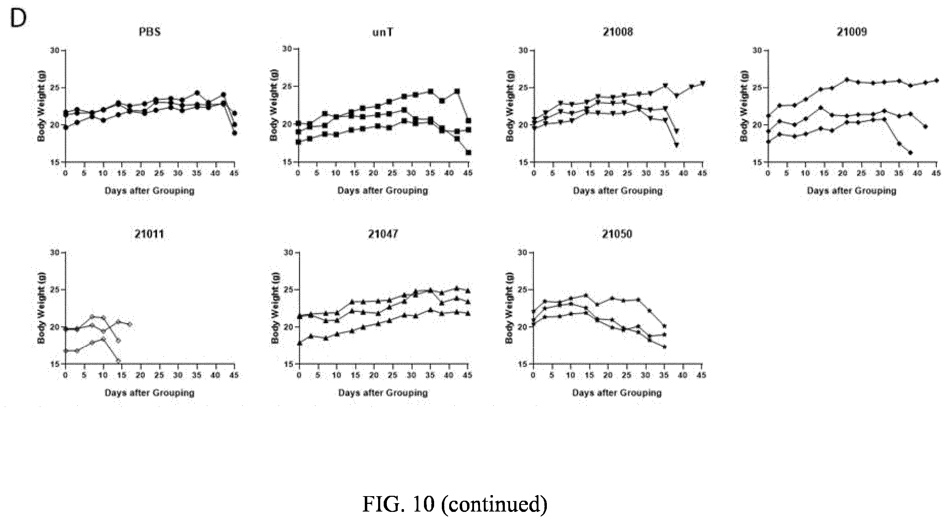

In order to study the ability of five CAR-T cells, including 21047, to clear tumor cells *in vivo,* we constructed an NUGC4-luc cell subcutaneous graft tumor model based on NCG immunodeficient mice and studied the CAR-T efficacy therein. Specifically, 100 µL (5 × 10⁶ cells) of the human gastric cancer cell line NUGC4-luc was inoculated subcutaneously into the back of NCG mice using a 1 mL syringe to establish a subcutaneous graft tumor model. Once the tumors reached about 50-150 mm³, infusion of CAR-T cells was started. As shown in FIG. 10, the mice of different groups were each infused with 1 × 10⁶ CAR-T cells or untransduced T cells (unT). Subsequently, the tumor growth condition, body weight and health status of the mice were continuously monitored. Finally, mouse subcutaneous tumor growth curves and mouse body weight growth curves (mean ± s.e.m.) were plotted using the Graph Pad Prism7.0 plotting software. The results are shown in FIG. 10A. At the end of the study, both molecules 21050 and 21047 achieved complete tumor clearance, while neither molecule 21008 nor 21009 achieved complete tumor clearance. We further compared tumor sizes on D35, with the order being 21050 < 21047 < 21009 ≈ 21008 (FIG. 10B). Additionally, the tumor sizes in the 21008 and 21009 treatment groups showed no significant difference from the unT group, indicating relatively weak efficacy (FIG. 10B). During the study, there was no significant change in body weight in the 21047 treatment group, while one or more mice in each of the 21008, 21009, 21011, and 21050 groups experienced significant weight loss, deterioration in status, or death (FIGs. 10C and 10D). The above results indicate that, compared to other molecules, 21047 had a significant safety advantage, while also demonstrating significant efficacy.

### 5. In vitro safety study of CLDN18.2 CAR-T

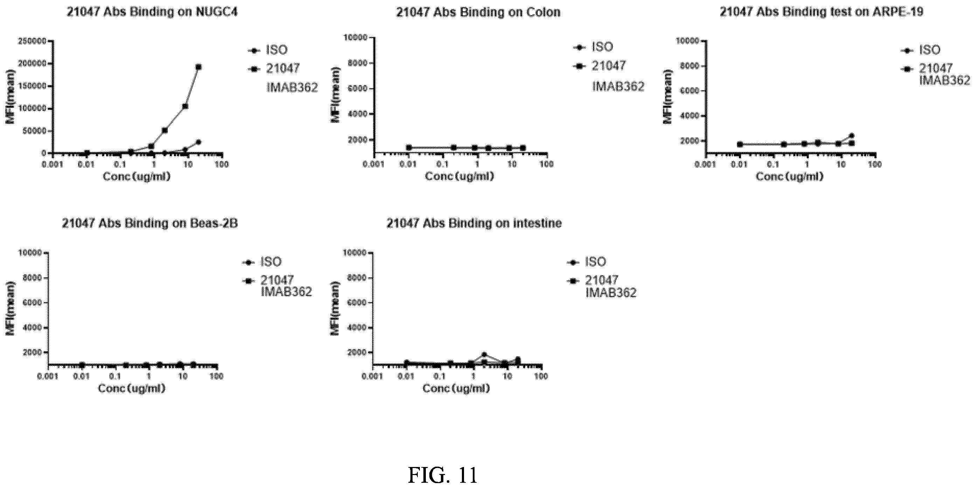

Further, in order to verify the non-specific binding of 21047 to primary cells, we diluted 21047 to different concentrations and incubated it with CLDN18.2-positive target cells NUGC4 or primary cells (lines), and then performed flow cytometry to detect MFI, with IMAB362 as a positive control antibody and an isotype as a negative control antibody. The results are shown in FIG. 11, indicating that both 21047 and IMAB362 bound normally to CLDN18.2-positive target cells NUGC4, but did not bind to primary cells such as colon, ARPE-19, Beas-2B, and Intestine.

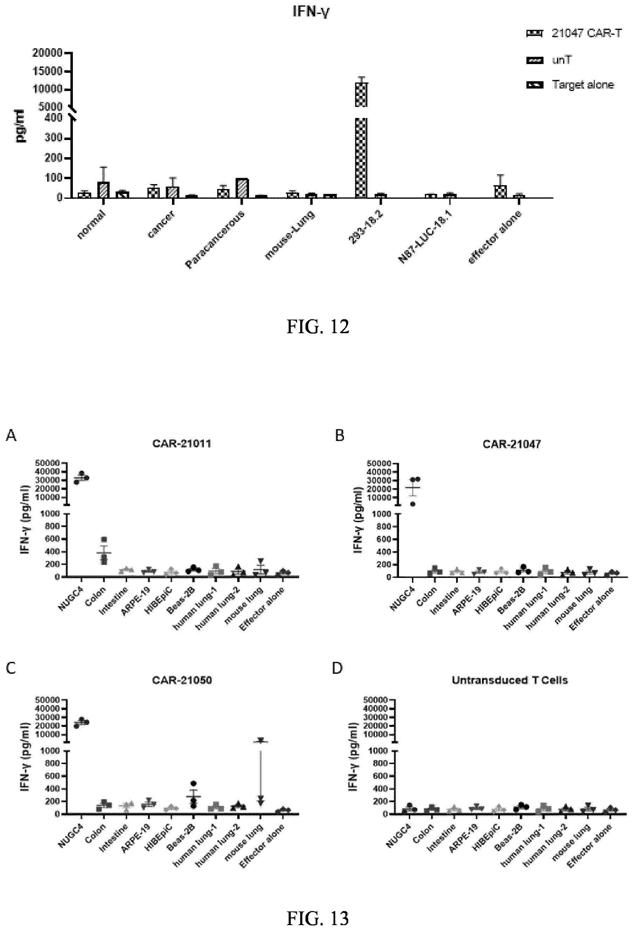

Since lung tissue cells express CLDN18.1, in order to study whether CLDN18.2 CAR-T is toxic to lung tissues, we studied its cytokine-releasing ability when killing human lung tissues, lung cancer, paracancerous tissues, and mouse lung tissues. The results are shown in FIG. 12. 21047 CAR-T cells did not show a significant increase in IFN-γ secretion levels after co-incubation with human and mouse lung tissues, while IFN-γ secretion levels reached up to above 10000 pg/mL in the case of co-incubation with 293-CLDN18.2 cells. As a negative control, in the case of co-incubation with N87-luc-18.1, there was no significant increase in the IFN-γ secretion levels. The above results indicate that 21047 CAR-T had no significant toxicity to lung tissues.

Further, in order to verify the safety of 21047 CAR-T, we analyzed the killing ability of 21047 and other CAR-T cells prepared from PBMCs of three different donors against primary human tissues/cells (lines), including Beas-2B (primary human lung cell line), ARPE-19 (human retinal epithelial cells), primary colon cells (primary human colon cells), HIBEpiC (human intrahepatic biliary epithelial cells), primary intestine cells (primary human small intestine cells), human lung tissues, and mouse lung tissues. The results shown in FIGs. 13A/C indicate that after co-incubation with the primary tissues/cells (lines), 21011 and 21050 CAR-T cells exhibited a significant increase in IFN-γ in some cases (such as the colon cells or primary lung cells), suggesting that 21011 and 21050 might exhibit potential tissue toxicity, etc. The results shown in FIGs. 13B/D indicate that 21047 CAR-T and unT cells did not show a significant increase in IFN-γ secretion levels after co-incubation with the primary human cells (lines), while IFN-γ secretion levels reached up to above 5000 pg/mL in the case of co-incubation with CLDN18.2-positive cells NUGC4. The above results show that 21047 CAR-T cells did not exhibit non-specific binding and activation to human lung, eye, colon, small intestine and intrahepatic biliary epithelial cells, indicating good safety.

### 6. In vitro functional study of CLDN18.2 CAR-T with different signaling domains

In order to study the impact of different combinations of hinge regions, transmembrane regions, and intracellular signaling domains on CAR-T function, we further constructed four different combinations of hinge regions, transmembrane regions, and intracellular domains based on 21047, the structures of which are shown in the following table:

| Molecule ID | Structure | | | |
|---|---|---|---|---|
| | Hinge region (H) | Transmembrane region (TM) | Co-stimulatory domain | CD3ζ |
| 210471ib-33B | CD28-H | CD28-TM | CD28 | CD3ζ |
| 210471ib-35B | CD8-H | CD8-TM | OX40 | CD3ζ |
| 210471ib-41B | short CD28-H | CD28-TM | 4-1BB | CD3ζ |
| 210471ib-42B | CD8-H | CD8-TM | ICOS | CD3ζ |

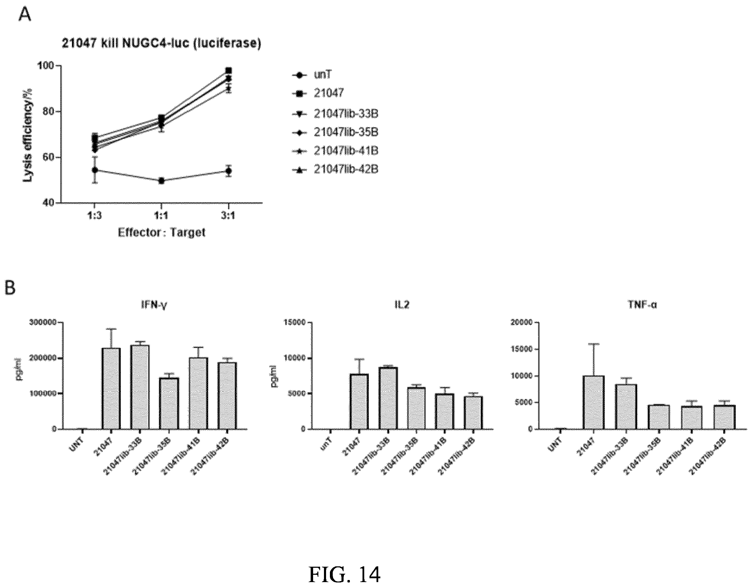

The effects of different structures on the killing ability against target cells and the cytokine secretion after target cell killing were compared *in vitro.* The results are shown in FIG 14, indicating that CAR-T with different hinge region, transmembrane region and co-stimulatory domain structures could all kill positive target cells and normally secrete cytokines such as IL2 and IFN-γ. The molecules such as 21047lib-35B (CD8-OX40-CD3ζ structure), 21047lib-41B (CD28-41BB-CD3ζ structure), and 21047lib-42B (CD8-ICOS-CD3ζ structure) showed no significant difference in their ability to kill target cells and secrete cytokines as compared to the original 21047 (CD28-CD3ζ structure).

The above examples only illustrate several embodiments of the present disclosure for the purpose of specific and detailed description, but should not be construed as limiting the scope of the present disclosure. It should be noted that various modifications and improvements can be made by those of ordinary skill in the art without departing from the spirit of the present disclosure, and such modifications and improvements shall fall within the protection scope of the present disclosure. Therefore, the protection scope of the present disclosure is determined by the claims, and the specification and the accompanying drawings can be used to illustrate the content of the claims.

## Claims

1. A chimeric antigen receptor comprising an extracellular domain comprising a CLDN18.2 single domain antibody, wherein amino acid sequences of heavy chain complementarity-determining regions of the single domain antibody are set forth in SEQ ID NOs: 1-3.

2. The chimeric antigen receptor according to claim 1, wherein the single domain antibody is humanized.

3. The chimeric antigen receptor according to claim 2, wherein an amino acid sequence of the single domain antibody is set forth in SEQ ID NO: 4.

4. The chimeric antigen receptor according to any one of claims 1-3, comprising a hinge region, a transmembrane region, and an intracellular signaling region.

5. The chimeric antigen receptor according to claim 4, wherein the hinge region is selected from a hinge region of CD8, CD28, IgG1, IgG4, 4-1BB, ICOS, OX40, CD40, CD80, or CD7, or a CH3 or CH2-CH3 constant region.

6. The chimeric antigen receptor according to claim 4, wherein the transmembrane region is selected from a transmembrane region of CD8a, CD28, CD4, ICOS, CD7, CD2, CD80, CD40, OX40, CD27, LFA-1, 4-1BB, ICOS, CD3ζ, or CD3ε.

7. The chimeric antigen receptor according to claim 4, wherein the intracellular signaling region comprises a CD3ζ signaling domain.

8. The chimeric antigen receptor according to claim 4, wherein the intracellular signaling region further comprises one or more selected from the following proteins or intracellular signaling regions thereof: CD28, 4-1BB, OX40, ICOS, CD27, MYD88, KIR2DS2, DAP10, DAP12, CD3ζ, TLRs, CD2, LFA-1, CD8α, CD40, CD80, and CD3ε.

9. The chimeric antigen receptor according to claim 4, wherein an amino acid sequence of the chimeric antigen receptor is set forth in SEQ ID NO: 5.

10. An isolated nucleic acid capable of expressing the chimeric antigen receptor according to any one of claims 1-9.

11. A vector comprising the nucleic acid according to claim 10.

12. An immune cell expressing the chimeric antigen receptor according to any one of claims 1-9.

13. The immune cell according to claim 12, wherein the immune cell is selected from a T cell, a B cell, an NK cell, a macrophage, and a dendritic cell.

14. The immune cell according to claim 13, wherein the T cell is selected from a helper T cell, a cytotoxic T cell, a memory T cell, a regulatory T cell, a MAIT cell, an NKT cell, and a γδ T cell.

15. A pharmaceutical composition comprising the immune cell according to any one of claims 12-14.

16. Use of the immune cell according to any one of claims 12-14 in the preparation of a medicament for killing CLDN18.2-positive tumor cells.

17. The use according to claim 16, wherein the medicament is used for preventing or treating gastric cancer, pancreatic cancer, or esophageal cancer.
